# EUROPEAN PATENT SPECIFICATION

(11) **EP 4 570 221 B1**
(45) Date of publication and mention of the grant of the patent: **18.03.2026**
(21) Application number: 24202224.2
(22) Date of filing: 24.09.2024
(51) Int. Cl.: A61F 13/15

(54) **PRODUCTION LINE FOR THE MANUFACTURE OF SANITARY PRODUCTS SUCH AS ABSORBENT PRODUCTS FOR SANITARY USE**
PRODUKTIONSLINIE ZUR HERSTELLUNG VON SANITÄRPRODUKTEN WIE ABSORBIERENDEN PRODUKTEN FÜR DEN SANITÄRGEBRAUCH
CHAÎNE DE PRODUCTION POUR LA FABRICATION DE PRODUITS SANITAIRES TELS QUE DES PRODUITS ABSORBANTS À USAGE SANITAIRE

(30) Priority: 15.12.2023 IT 202300026757
(43) Date of publication of application: 18.06.2025
(73) Proprietor: GDM S.P.A., 40133 Bologna (IT)
(72) Inventor: PIANTONI, Matteo, 41042 Fiorano Modenese (IT); ZAVALLONI, Alessandro, 40133 Bologna (IT); CARUANA, Paolo, 40133 Bologna (IT)
(74) Representative: Emmi, Mario

(56) References cited:
- US-A1- 2013 014 902
- US-A1- 2015 173 965
- US-A1- 2015 176 750

## Description

### Scope of the invention

The present invention concerns the technical field regarding a production line for realizing sanitary products, preferably wearable sanitary products such as for example pads for men, women or children, handkerchiefs for example for face use, etc., and/or in particular absorbent products for sanitary use.

In particular, the invention refers to a production line that, overall, is very flexible in its composition and/or realization, resulting at the same time in a structure that, in addition to being versatile, is also safe for the operators by guaranteeing safe access to its operative or control areas and further guaranteeing easy access to said areas.

### A brief outline of the prior art

The machines (or production line as it may be) for the production of sanitary products (for example absorbent products for sanitary use) have been well known for some time.

The sanitary products that can be realized are manifold and may include sanitary products that can be worn by a user, such as for example the products belonging to the category of disposable diapers, disposable pads, disposable sanitary pads, handkerchiefs, leakage protection mattress covers, etc.

Generally, these production lines are developed through manufacturing sections or manufacturing stations as it may be (also defined herein with the term of manufacturing units or simply units) that are connected to each other in succession. In this way, sanitary products can be realized by starting even from supplying, to the production line, raw materials and then obtaining the finished and even packaged product coming out of the line.

For example, in the case of sanitary products such as the category of the disposable diapers (or similar), it is started by processing the cellulose that forms the "absorbent core" of the product and which is trapped between the two sheets of material that is generated by unwinding the master reels. The product is made through manufacturing steps that are carried out in succession in the various manufacturing stations (or units as it may be as said) until the finished product is completed, which can even be packaged at the end of the forming process thereof.

That being said, it is known that in the technical sector of production lines for the realization of sanitary products, in particular absorbent products for sanitary use, these production lines are made by the above introduced units that are connected to each other in succession. Each unit, actually, houses one or a series of operative apparatuses that is/are used to carry out a certain manufacturing step. The operative apparatus may for example provide a motor and an operative part which is driven by said motor to perform a certain operation. The operative part may for example comprise rollers that are driven in rotation to perform a certain manufacturing step on the product. According to the known technology, the unit therefore consists of a support frame to which coating panels are fixed. Each panel is configured to accommodate a certain operative apparatus (one or more than one), such that the operative apparatus(es) is/are permanently fixed to the panel forming a whole with it (defined as an operative assembly).

In particular, the operative apparatus can comprise a motorization and an operative part and therefore the operative part emerges from a face of the panel and the motorization emerges from the opposite face. The panel with the operative apparatus connected in a removable way to it thus becomes a whole (i.e. an operative assembly or operative group) that can easily be detached from the unit frame and reapplied or applied to it in general.

In this way there is great versatility and ease of intervention since, in case of replacement of an operative apparatus, the entire operative assembly (or operative group as it may be) made from the panel with the apparatus connected to it can be removed directly and replaced with another operative assembly already previously prepared. This can be useful not only during failure but also if, for example, it is decided to modify the machine, so that for example manufacturing with different formats is allowed. In this simple way, the panels with the operative apparatuses connected to them can be removed and replaced with other panels of the same dimension, previously prepared and provided with new operative apparatuses (for example operative apparatuses of different dimension and/or power).

In addition, again in accordance with a further known technique, it is possible to connect the panels to the frame of the unit in such a way that they are separated from each other by a "gap" of distance in such a way that they result in mutually independent elements or, in other words, such that a panel placed below another panel does not have to support the weight of the panel placed above. This allows the panels to be removed completely independently of each other as none of them supports another panel.

Having said that, however, the prior art is not entirely conclusive in terms of the technical problem related to the flexibility of the production line in its construction/design and management.

The manufacturing units that are connected in succession to each other are often made specifically for the specific line.

This implies that a unit is not insertable in a different line.

In addition, also the type of panels that a unit can accommodate is a type of panels of a specific size. Each production line will therefore have its own units specially made to connect to each other with the coating panels equipped with the relative operative apparatuses that are specific to that line and cannot be used for other lines.

This therefore makes the construction of the lines expensive as each unit is not interchangeable with other units. Moreover, the same lines are not further flexible as parts used for one line can hardly be adapted to other lines, not allowing an interchangeability of components. In other words, the units of a specific line can hardly be used and inserted in another line.

In the same way, the panels of a unit relative to a line can be hardly used for units of other lines.

This results in a low flexibility of the current production lines.

In addition, automated robots are known and used to perform cleaning operations, such as for example extraction of the dust that is generated during the production process of the article or product in general.

These robots can be mobile, for example on guides obtained in the units themselves.

It is clear that, in accordance with the prior art introduced above, an automated robot for mobile cleaning along the operative units of a production line is hardly adaptable to a unit of another line, making the current lines inflexible as already mentioned. In particular, the interposition of a manufacturing unit inserted in another line can interrupt the continuity path for the robot.

Furthermore, the overall layout of the production lines is such that the protections applied for the safety of the operators are bulky and specific to the specific production line, greatly reducing the manoeuvring space for the operators, making the cleaning operation with the appropriate robots or machinery in general harder and not allowing an adaptation of these protections to different lines. At the end of the operational life of the production line, therefore, parts such as protections and any robots, as well as the same apparatuses connected to the panels, will hardly be reusable for other lines.

Document US2015/173965 is also known.

### Summary of the invention

It is therefore an object of the present invention to provide a production line that at least partially solves the aforementioned technical drawbacks.

In particular, it is an object of the present invention to provide a production line for sanitary products (also called sanitary articles), in particular wearable sanitary products and/or absorbent products for sanitary use and/or absorbent products for sanitary use that is versatile, combinable in a modular way and therefore flexible.

More particularly, it is an object of the present invention to provide a solution in which the manufacturing units of the line are therefore replaceable with other manufacturing units coming also from other lines and in any case allowing in the same way that it can be continued with using, if present, the cleaning robot(s) in charge and maintaining the same safety protections that guarantee adequate handling space and at the same time safety.

These and other purposes are achieved with the production line, for example for absorbent products for sanitary use, in accordance with claim 1.

The line comprises:
At least two or more manufacturing units (mod1, mod2, .. modn) arranged in succession to each other for forming said production line.

Each manufacturing unit comprises at least one or more panels (11).

Each panel (11) is equipped with one or more operative apparatuses (60) connectable to the panel (11) in a removable way for executing at least one manufacturing process along said production line.

In this way, the line can perform its manufacturing operations.

Furthermore, each manufacturing unit (mod1, mod2, ..modn) comprises at least one structural support frame (52, 53, 54) to which the at least one or more panels (11) are connected in a removable way.

More particularly, advantageously, the set of one or more operative apparatuses (60) connected to a panel forms an operative assembly (11, 60).

In this way, advantageously, an entire operative assembly (11, 60) can, in use, be removed and reconnected to a structural support frame (52, 53, 54) and/or a different operative assembly can be connected to a structural support frame in replacement of the previous removed operative assembly.

Furthermore, advantageously, there could be several operative apparatuses for each structural frame and/or, vice versa, there could be several structural frame for a single operative apparatus.

In accordance with the invention, said at least one structural support frame (52, 53, 54) of said at least two or more manufacturing units (mod1, mod2,..modn) is of standard type such that each production line can be obtained by combining in a modular way together, according to any combination, any number of manufacturing units (mod1, mod2,..modn) each comprising said at least one standardized structural support frame (52, 53, 54).

Thanks to this standardization solution of the structural support frame (52, 53, 54), the intended purposes are thus achieved.

In particular, each production line can now be obtained by combining together any number of standard manufacturing units, so that any combination can be made according to the needs.

The standardization of the structural support frame (52, 53, 54) means, therefore, that these frames actually belong to a group of frames having characteristics of compatibility with each other, thus allowing them to be freely combined.

In this way, advantageously, each manufacturing unit actually belongs to a standard group and obviously all of them are compatible, therefore combinable between them.

In particular, the realization of standard manufacturing units as described above (therefore in particular through the realization of standard structural support frames) makes the realization of the production lines flexible since each unit constitutes a standard module, that is, belonging to a predefined group and therefore compatible with the other modules of the group, although the other modules of the group have different frame configurations (for example, geometric ones of the frame) (for example, there may be frames with a different overall shape and/or longer and/or taller parts and/or in any case having differences).

In any case, the structural support frame will have parts for connection to another structural support frame of another unit with geometric compatibility characteristics.

It is therefore possible, in absolute flexibility, to realize production lines in which the various manufacturing units of the line are all with the same configuration or lines can be realized by combining between them standard modules always belonging to said standardized group but with at least two or more of said standard modules having a frame configuration different from each other but obviously belonging to the standard group.

This therefore derives from a standardized design in which a large number of standard units of the group guarantee, through their infinite combinations, the realization of infinite different lines.

In a non-limiting example, the group of units may for example consist of three manufacturing units (unit 1, unit 2, unit 3) different from each other (for example geometrically as the standard frames of each one are geometrically different from each other but compatible) and designed so that they can cover the cases of possible needs. Exactly according to the concept of "Lego" bricks, they can be combined together realizing multiple production line combinations. A production line could for example provide a number of units 1 connected to each other in succession to which a unit 2 is connected which is followed by a further unit 1, just to give an example. The combinations would be infinite, also allowing certain units to be reused as replacement on other lines that use units of the same group.

The design would therefore be limited to a repetitiveness of the same units provided for the group, realizing a certain number that could also be stored in warehouses and ready for delivery in order to combine them quickly for the realization of the line.

Each of these units would be structured, for example, so that it can accommodate an automatic cleaning robot, thus making the proposed solution versatile even when a module is transferred to another production line to replace a previous module.

According to the invention and following tests and experiments, the Applicant has found that said panels are also be part of a group of standard panels each having a predetermined configuration different from the other panels of said group.

In this manner, said standard panels of the group are, in turn, compatible with the standard frames belonging to said group of manufacturing units.

In this way, advantageously, any combination of standard panels belonging to said group of standard panels can be applied in a removable way to each of said standard manufacturing units.

More particularly, even for the panels applicable to these units introduced above, there will be a condition in which panels with the same configuration will be usable with each other and/or it will be possible to combine together standard panels always belonging to said group but in which at least two of which are with configuration different from each other.

It is therefore clear that this solution provides high flexibility.

This ensures that there are multiple combinations of panellings applicable to units different from each other.

Advantageously, said standard frame configuration is a geometric configuration.

This implies, therefore, that the standard structural support frames of said (standard) units can differ from each other in the geometries (e.g. dimensions).

Advantageously, in the same way, said configuration of the standard panels can be a geometric type configuration.

This implies, therefore, that the standard panels belonging to the group can differ from each other in the geometries (e.g. dimensions).

Therefore, in terms of design, "n" types of units with standard structural support frame and "m" types of panels can be provided, with the "n" units being compatible with each other and the "m" panels compatible with said "n" units.

In this way, in terms of design, large numbers of manufacturing units can be realized with such units that are all standard (therefore standard frames) and such as to allow a construction of the line by combining these units of the group with each other according to any order.

The standard and/or non-standard panels with the relative operative apparatuses will be applied to them, according to any order.

In this way, replacements, repairs, etc. can be carried out quickly without long machine downtime as an entire operative assembly (panel + operative apparatus) can be prepared in advance and stored in the warehouse ready to be used for replacement purposes, for example.

Advantageously, at least part of the electronic componentry and/or of the hydraulic componentry and/or of pneumatic componentry can be placed at said panel (11) and/or on said at least one structural support frame (52, 53, 54).

This results in compact construction solutions.

As already anticipated above, advantageously, said standardization of the at least one structural support frame (52, 53, 54) can be a geometric standardization.

As already anticipated above, advantageously said production line can comprise a combination of said at least two manufacturing units (mod1, mod2,.. modn) equal to each other all having the same standard structural support frame (52, 53, 54).

Alternatively, advantageously the line may comprise said at least two manufacturing units (mod1, mod2, .. modn) different from each other and each having said at least one structural support frame (52, 53, 54) different from each other but always of standard type.

The high flexibility of the solution is evident from the above.

As already stated above, advantageously, said at least one or more panels (11) can be chosen between standard and/or non-standard.

This increases the versatility of the line in its realization phase.

Advantageously, said two or more manufacturing units (mod1, mod2,.. modn) can be configured to allow applying a protective barrier (Ps1, Ps2, .. Psn) along the direction of longitudinal development of said production line.

In this way, advantageously, a protection is created.

According to one aspect of the invention, said protective barrier (Ps1, Ps2, .. Psn) can advantageously be placed at a distance from the front part (54) of the production line in such a way as to form an operative corridor 100 that an operator and/or a further machinery can access and/or enter.

In this way, a safe passage path is created that also a robot can access.

More particularly, advantageously, in this way there is the dual advantage of having an easy space for the operator and for any one or more robots provided.

Advantageously, the protective barrier can be fixed to a part of the structural support frame (52, 53, 54) of said manufacturing unit (mod1, mod2,.. modn) and/or to the ground.

Advantageously, the barrier being spaced from the units of the line and applied to a standardized support frame, this makes this barrier flexibly applicable to any combination of units.

Advantageously, in all the configurations described, this protective barrier (Ps1, Ps2, .. Psn) can be made up by a plurality of panels (Ps1, Ps2, Psn) placed in sliding succession to each other between a packed position, in which they overlap one or more preceding panels for generating an opening, and an extended position in which they close said opening at least partially.

In this way, advantageously, it is possible to create an opening that can affect the entire length of the line itself.

More particularly, thanks to this sliding solution, when the barrier protects the entire length of the line longitudinally, it is actually possible, by sliding all the panels, to generate an opening that covers the entire longitudinal length of the line.

Advantageously, said panels (Ps1, Ps2, .. Psn) can be made of plastic and/or transparent material to allow seeing through them.

Advantageously, an automated robot (110) can therefore be provided.

Advantageously, said robot 110 can therefore be configured to clean at least one of said at least two or more manufacturing units (mod1, mod2,..modn) of the production line, along the production line.

Advantageously, said automated robot (110) can for example be placed at the operative corridor 100 delimited by said protective barrier (Ps1, Ps2, ..Psn).

In this way it can operate safely.

In particular, the operative units can provide a track that continues along the guides when these are coupled.

The track forms the guide and the robot is constrained to move along said guide.

Advantageously, therefore, the standardization of the units (in particular the frame as said) makes it possible to use the robot even in cases of replacement of the units with others, for example during an upgrading of the line.

Advantageously, the robot (110) can be movable along a guide obtained near said two or more manufacturing units (mod1, mod2, ..modn).

Advantageously, said production line may comprise one or more electrical panels (120) and relative electrical wirings.

These electrical panels (120) can be placed at a certain distance from said two or more manufacturing units (mod1, mod2,.. modn) along the direction of manufacturing development of said production line at a certain distance from the rear face (52) of the production line.

In this way, advantageously, a second passage corridor (130) is formed which is delimited by said one or more electrical panels (120) and by the rear face (52) of the production line.

The Applicant has advantageously found that this configuration facilitates the access and operation of the operators to/at the electrical panels, compared to the prior art in which they are placed on the roof of the line.

Advantageously, the electrical wirings of the one or more electrical panels (120) are covered by raceways (140) or walkable humps (140).

This also contributes to safety.

A further object of the present invention is also the use of a production line according to one or more of the above characteristics for the realization of absorbent products for sanitary use.

A further object of the present invention is a method for realizing a production line adapted to realize absorbent products for sanitary use, in accordance with the claim 13.

In accordance with the invention, the method comprises a step of realizing said production line by assembling at least two or more manufacturing units (mod1, mod2, .. modn) in succession to each other in accordance with a predetermined combination and fitting said at least two or more manufacturing units (mod1, mod2,.. modn) with one or more relative panels (11) equipped with one or more operative apparatuses (60) for executing at least one manufacturing process along said production line.

According to the invention, said step of realization comprises a subphase of equipping said two or more manufacturing units (mod1, mod2,.. modn) with at least one structural support frame (52, 53, 54) of standard type.

In this way, advantageously, each production line is obtainable by combining in a modular way together, according to any combination, any number of manufacturing units of said two or more manufacturing units (mod1, mod2, .. modn) each having said at least one standardized structural support frame (52, 53, 54).

This therefore gives great versatility.

Advantageously, in case of need of modification of said production line, it can be provided for the step of removing one or more manufacturing units of said two or more manufacturing units (mod1, mod2,..modn) and the relative replacement with one or more manufacturing units (mod1, mod2, ..modn) each comprising at least one standardized structural support frame (52, 53, 54).

### Brief description of the drawings

Further features and advantages of the present production line, according to the invention, will become clearer with the following description of some of its embodiments, made by way of example and not limitation, with reference to the accompanying drawings, wherein:
- figure 1 schematically shows a group of standard modules (therefore with a standardized structural support frame) that can be combined together according to any combination for forming a production line for sanitary products, in particular absorbent pads for sanitary use;
- figure 2 shows an example of a first modular production line 1 formed by a certain succession of units each one constituting a module of the group; the figure shows a second modular production line in which, by way of non-limiting example, it is highlighted how the module 2 of said second production line can be replaced with the module 1 of the first production line or with a new module **3;**
- figure 3 shows a group of standard panels that can be combined together according to any combination when applied to one of the units of the group described in figure 1;
- figure 4 schematically shows a unit in side view equipped with a frame to which two panels are connected, by way of example, one on top of the other and with each panel supporting an operative apparatus connected to it for forming an operative assembly;
- figure 5 is an overall view of an example of a production line in accordance with the invention in which, frontally, the protection panels are highlighted;
- figure 6 shows a portion of line with a sliding protection panel in open position to highlight the interior where a portion of panels and a cleaning robot can be seen;
- figure 7 further shows from above the production line of figure 6 highlighting how, in accordance with the described solution, the electrical panels are placed side-by-side to the production line from the rear part of the line and with the raceways for the passage of cables; in this way a passage corridor is formed;
- The sequence of figures from 8A to 8D shows the type of protection used that provides panels (for example of transparent plastic material) slidably mounted on a frame in such a way that they can be packed by overlapping each other and thus ensuring the possibility of creating a continuous access that can include the substantial length of the entire line.

### Description of some preferred embodiments

The present invention is now described in detail with reference to the accompanying figures.

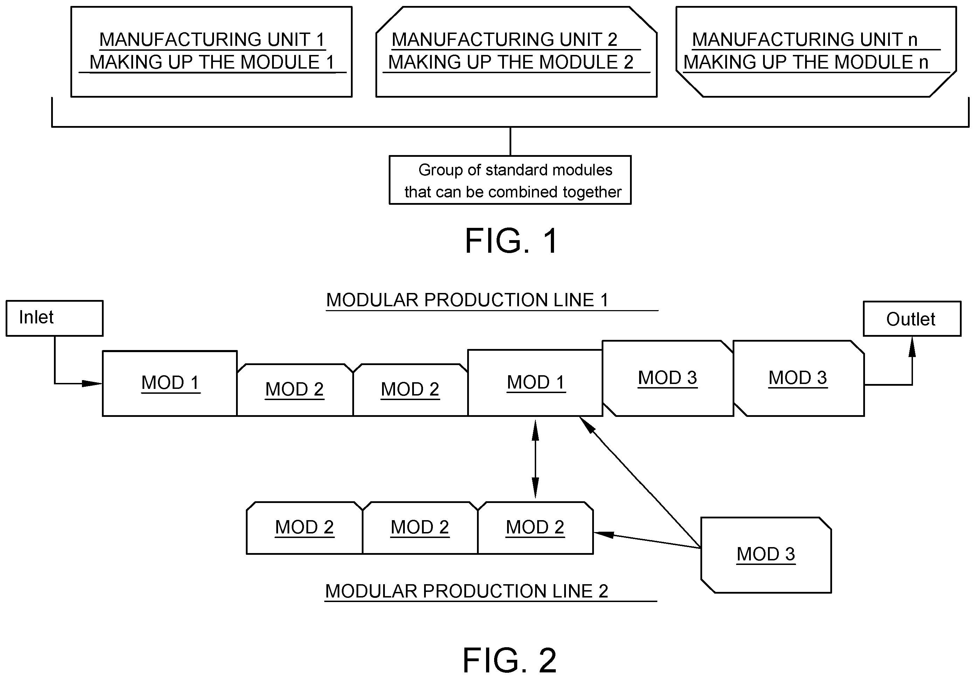

Figure 1 shows a group of modular and standard manufacturing units (also called manufacturing stations).

Modularity means that they are modular, that is, compatible with each other and therefore can be connected and/or combined together in succession for forming a production line.

They are also standard, which means that a certain number of modules (therefore units) of predefined configuration, generally different from the other modules of the same group, which allows a free combination thereof in succession to each other, are provided in the modular group.

As mentioned, since they are modular they are compatible and combinable together according to any order.

As shown in the non-limiting example of figure 1, three types of units (replicable of course in any number), i.e. three models of units, are represented by way of non-limiting example. Obviously, the group can be formed by any number of types of units.

Each unit can be different from the others of the group in terms of dimension and/or generic conformation and/or materials, etc. and therefore be actually different from the others, for example, even only in terms of geometric differences (therefore dimensions and/or shapes in general).

By way of example, figure 1 shows three different shapes with different dimensions. Thus, the group consists of three models different from each other in the geometry. Each module is therefore standard in terms of design and any number thereof of each module can be realized. In this way, since they are combinable together according to any combination, with the realization of the aforementioned group it is possible to combine together the units realizing standardized production lines but very varied between them.

By way of example, figure 2 shows a production line that consists of a number of six modular units connected to each other in succession. They are part of the modules of the standard group of the example indicated in figure 1 and therefore there are in succession:

A modular unit 1 which is followed by two modular units 2 which are followed by a further modular unit 1 and which is followed by two further modular units 3. The figure then schematically shows a manufacturing inlet to the line and an outlet of the finished sanitary product.

Now, as already anticipated, the modular units are standard, that is, a certain type of models constituting a group being defined, the lines are realized by combining together the only models of the group according to the needs. Each unit is therefore compatible with all the others in the group in terms of connections and to determine a continuity.

As therefore indicated in figure 2, the example of a modular production line 2 is reported which, by way of example, consists of only three units of type 2.

In accordance with this solution, it is therefore possible to add or remove modular units with extreme ease as these are all standard and compatible with each other. For example, as indicated in figure 2, the unit 1 of the first line can be inserted in place of the unit 2 of the second line should this be necessary, just as a new unit 3 can replace a unit 1 or a unit 2 of the first and second line, respectively.

In particular, therefore, the modular units are all compatible with each other and it is possible to add and/or remove and/or replace a unit from/in a line according to the needs.

If, for example, with reference to figure 2 it is necessary to modify a manufacturing format in the modular production line 2, it is then possible, for example, to replace the relative unit 2 with a new unit 3, provided that it belongs to the standard group provided.

In this way, the design is also simple as the modules (or units as it may be) realized will always be the same relative to the group and then, by combining the modules together, it will be possible to realize lines of any length and for any need with great flexibility and versatility of the line itself.

Thus, for example, if it is decided to realize a group made of three standard models of units, it will be possible to realize and store a large number of such models of the group in order to realize the required lines by combining these units of the group together.

More particularly, as can also be seen from figure 1 and figure 2, what is standardized in the module to allow such free combination is the structural support frame (52, 53, 54) of each module.

Each module will therefore have a structural support frame that belongs to a standardized group, making it possible to achieve the flexibility and interchangeability described above.

Each frame can therefore have geometric characteristics that are different from module to module while still having mutually compatible connection parts that ensure that several modules can be freely arranged in succession according to the needs.

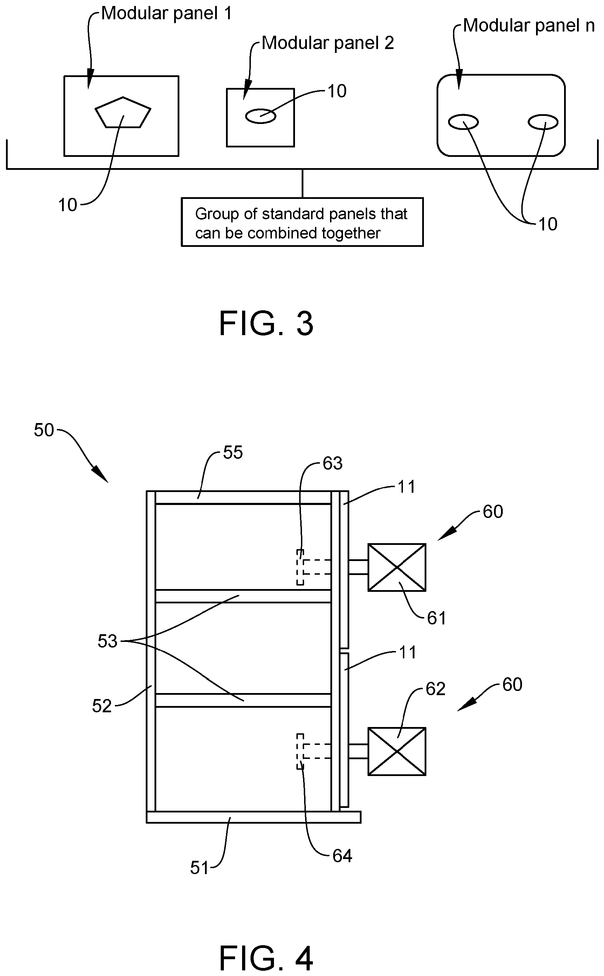

Going to figure 3, according to a further configuration of the invention and without prejudice to what has been described, the same concept can be further and advantageously applied also to the panels.

More in particular, as also known in the state of the art, the units described herein comprise the structural support frame (also simply called support frame or frame) which is as said standard.

In this regard, figure 4 shows a modular manufacturing unit 50 having a rest base 51 on which the structural support frame (52, 53, 54) raises. Said support frame may provide one or more rear vertical bars 52 and one or more front vertical bars 54, preferably arranged substantially parallel to each other.

In this way, a front face 54 and a rear face 52 of the unit are formed.

Said bars raise orthogonally from the base 51.

Finally, an upper closure 55 may be provided which may also be a single beam or a combination of beams and/or a panel.

As mentioned, the geometries of this frame (reported above in a non-limiting example) may vary from module to module but are included in a standardized group.

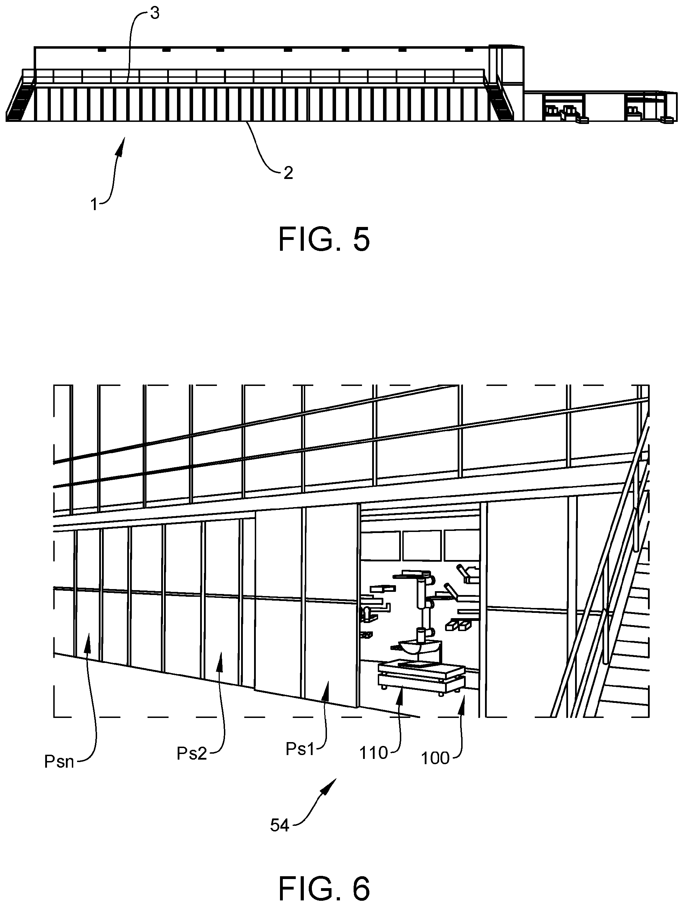

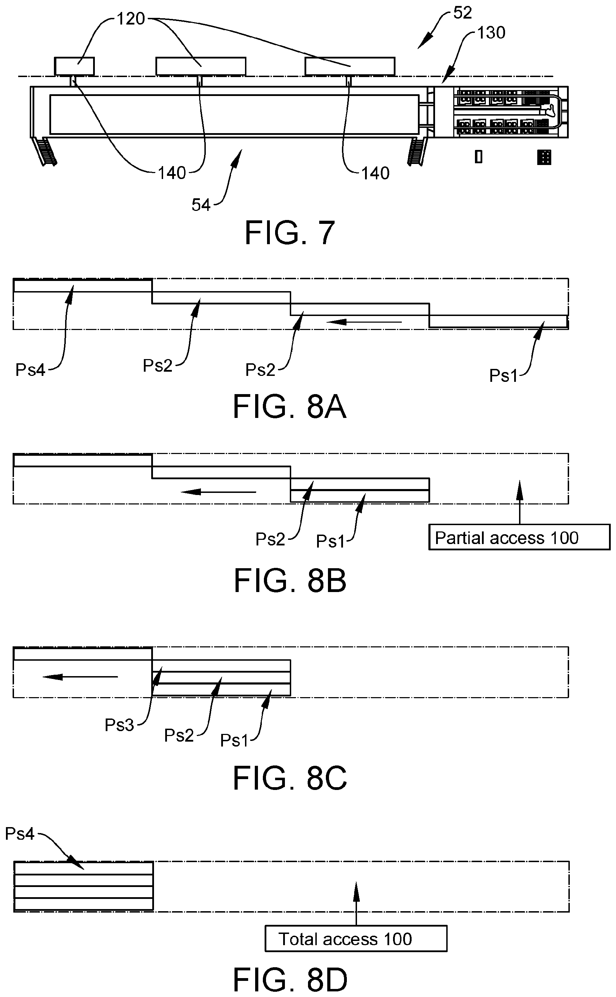

With reference to figures 6 and 7, the front face 54 of the units conforms the front face of the line and the rear face 52 of the unit (relative to the trusses 52) conforms the rear face of the line.

With reference to figure 4, on the front face (trusses 54) there are applied the panels 11 fitting the operative apparatuses 60 integral with them, for forming the so-called operative assemblies (11, 60).

An operative assembly is therefore the set of the panel 11 to which one or more operative apparatuses 60 are fixed in a removable way.

With reference to figure 4, therefore, an operative apparatus 60 formed by an operative part (61, 62) and a motorization part (63, 64) is connected to a panel 11.

The motorization part, with reference to figure 4, is the one indicated with numberings 63 and 64 and are placed inside the structure of the unit whereas externally there is the operative part performing the manufacturing and indicated in figure 4 with numberings 61 and 62. To each panel, therefore, these operative apparatuses are connected in a removable way such that there is an operative part (61, 62) that stretches from one face of the panel and a motorization part that, on the opposite part, extends from the other opposite face.

The panels are arranged so that the operative apparatuses can be fixed thereto and, as for example schematized in figure 3, special passage holes 10 of different conformation can be provided depending on the operative apparatus to be connected. The connection can be completed with appropriate connection means such as screws, bolts, etc.

The panel can then, in turn, be easily connected to the frame in a removable way, for example always through connection means such as screws, bolts, etc.

According to a further aspect of the invention, as schematized in figure 3, also the panels can advantageously be standard and therefore can be part of a group of standard panels that can be combined and compatible between them with all the modular units introduced above.

Also these panels are predefined models with conformations different from each other within the group.

The example of figure 3 shows, in a non-limiting way, three panels (three models that make up the predefined group) that are geometrically different from each other.

In this way it is possible to prepare a large number of operative assemblies (i.e. standard panel with operative apparatus) that are all compatible with each other with the units and can be connected together according to various combinations.

This increases versatility in that an operative assembly of a modular unit relative to a production line can for example be transferred to another unit of another line.

Likewise, in the same unit, an operative assembly can be replaced with an equivalent one already ready, for example in the event of a failure or after upgrading the line, for example by replacing this operative assembly with one already ready and compatible but of different configuration, for example mounting different motors and/or different apparatuses.

This provides a lot of versatility in the realization.

In any case, it is well understood that the line is very flexible even in the case of applying and/or using non-standardized panels.

According to a further aspect of the invention, each modular unit is realized as said in such a way as to be combinable with the other units of the group generating a continuity, albeit with a different configuration.

As highlighted in figure 6, this allows at least one cleaning robot 110 to be applied, which can for example move continuously along a track formed by the units combined together, and this guide track continues to be present even when a unit is replaced with one having a different configuration. All this because the units, as said, are standard and therefore are predefined models belonging to a group of models compatible with each other.

The robot 110 can be equipped with cameras and can possibly be driven remotely, as well as it could be programmed to be autonomous and automatic.

It has a cleaning end that acts to clean for example by suction and/or by spraying suitable substances.

The robot 110 moves within a corridor 100 from the front part 54, which part is delimited by the protections (Ps1....Psn).

These protections, always in accordance with a further aspect of the invention, are in the form of panellings or panels in general, for example of plastic and/or transparent material to allow seeing inside.

The protective panels, according to the invention, are mounted in a slidable manner so that they can be packed together.

Figure 5, which shows the entire line in front view, shows how these panels extend forming a protective barrier along the entire longitudinal length of the manufacturing line and, as can be seen from figure 6, spaced from the front part 54 of the line in such a way as to form a corridor of easy access for the operator.

These panels, as shown in the sequence of figures 8A to 8D, are all slidable between them along a direction of longitudinal development of the production line, so that they can be packed together.

This therefore allows, as schematized in figure 8A, to make the protection active or, as shown in the following sequences, to partially open an access opening by packing the panels in succession until an access aperture can be opened that can even involve the entire length of the line. This solution, therefore, unlike hinged doors, allows to create accesses of any dimension that can even involve the entire aperture or almost the entire aperture (longitudinal length) of the line itself.

It is also not necessary to start opening from the first panel Ps1 since the sliding system can also allow to slide, for example, only one panel Ps2 on PS3 and thus realizing the relative opening interposed in the barrier.

A special support frame, shown in the dotted line figures, allows the panels to be supported and have them slide as described, for example the same structural support frame of the module.

According to a further aspect of the invention, figure 7 shows the production line in top view.

According to the solution, the electrical control panels are now arranged on the ground from the rear part 52 of the production line (i.e. from the part towards which the motors 63, 64 extend, for example, with reference to figure 4) and the raceways or platforms 140 covering the electrical cables are highlighted. In this way, a further passage corridor 130 delimited by the electrical panels 120 and the rear face 52 is formed.

This solution, unlike the prior art in the specific sector, facilitates interventions on the electrical panels, guaranteeing an easy passage for the operator who, moreover, does not risk tripping over the various cables.

In use, therefore, a production line for absorbent products for sanitary use can be obtained by combining together the units belonging to a specific group, therefore by combining together only the designed and realized standard units.

In this way all the lines can be easily modified by adding or removing units belonging to said group.

Furthermore, advantageously, also the panels can possibly (not necessarily) be standard, and therefore they also belong to a predefined standard group, thus being compatible with said standard units.

It is therefore sufficient to build a large number of such units and/or panels by making them available, for example, in warehouses and then realize the lines by combining them together according to the needs.

Barriers and electrical panels, as described, are compatible with this modularity.

## Claims

1. A production line for absorbent products for sanitary use, said line comprising:
- At least two or more manufacturing units (mod1, mod2, ..modn) arranged in succession to each other for forming said production line,
- **Characterized in that:**
- Each manufacturing unit (mod1, mod2,..modn) comprises at least one or more panels (11), each panel (11) being equipped with one or more operative apparatuses (60) connectable to the panel (11) in a removable way for executing at least one manufacturing process along said production line, and wherein further each manufacturing unit (mod1, mod2, ..modn) comprises at least one structural support frame (52, 53, 54) to which the at least one or more panels (11) are connected in a removable way;
- said at least one structural support frame (52, 53, 54) of said at least two or more manufacturing units (mod1, mod2,..modn) is of standard type such that each production line can be obtained by combining in a modular way together, according to any combination, any number of manufacturing units (mod1, mod2,..modn) each comprising said at least one standardized structural support frame (52, 53, 54);
- And wherein said at least one or more panels (11) is/are standard so that said panels are part of a group of standard panels each having a predetermined configuration different from the other panels of said group, said standard panels of the group being compatible with the standardized structural support frames belonging to said manufacturing units so that any combination of standard panels belonging to said group of standard panels is applicable in a removable way to each of said manufacturing units.

2. The production line, according to claim 1, wherein at least part of the electronic componentry and/or of the hydraulic componentry and/or of pneumatic componentry is placed at said panel (11) and/or on said at least one structural support frame (52, 53, 54).

3. The production line, according to claim 1 or 2, wherein said standardization of the at least one structural support frame (52, 53, 54) is a geometric standardization.

4. The production line, according to one or more of the previous claims, which comprises a combination of said at least two manufacturing units (mod1, mod2, ..modn) equal to each other all having the same standard structural support frame (52, 53, 54) or, alternatively, comprises said at least two manufacturing units (mod1, mod2,..modn) different from each other and each having said at least one structural support frame (52, 53, 54) different from each other but always of standard type.

5. The production line, according to one or more of the previous claims, wherein said two or more manufacturing units (mod1, mod2, ..modn) are configured to allow applying a protective barrier (Ps1, Ps2, .. Psn) along the direction of longitudinal development of said production line, said protective barrier (Ps1, Ps2, .. Psn) being placed at a distance from the front part (54) of the production line in such a way as to form an operative corridor (100) that an operator and/or a further machinery can access and/or enter; preferably the protective barrier being fixed to a part of the structural support frame (52, 53, 54) of said manufacturing unit (mod1, mod2,..modn) and/or to the ground.

6. The production line, according to claim 5, wherein said protective barrier (Ps1, Ps2, .. Psn) is made up by a plurality of panels (Ps1, Ps2, Psn) placed in sliding succession to each other between a packed position, in which they overlap one or more preceding and/or subsequent panels for generating an opening, and an extended position in which they close said opening at least partially.

7. The production line, according to claim 5 or 6 wherein said panels (Ps1, Ps2, .. Psn) are made of plastic and/or transparent material to allow seeing through them.

8. The production line, according to one or more of the previous claims, comprising an automated robot (110) configured to clean at least one of said at least two or more manufacturing units (mod1, mod2,..modn) of the production line, along the production line.

9. The production line, according to claim 8, wherein said automated robot (110) is placed at the operative corridor (100) delimited by said protective barrier (Ps1, Ps2, ..Psn).

10. The production line, according to claim 8 or 9, wherein the robot (110) is movable along a guide obtained near said two or more manufacturing units (mod1, mod2,..modn).

11. The production line, according to one or more of the previous claims, comprising one or more electric panels (120) and relative electric wirings, said electric panels (120) being placed at a certain distance from said two or more manufacturing units (mod1, mod2, ..modn) along the direction of manufacturing development of said production line at a certain distance from the rear face (52) of the production line, in such a way as to form a second passage corridor (130) delimited by said one or more electric panels (120) and by the rear face (52) of the production line; preferably the electric wirings of the one or more electric panels (120) are covered by raceways (140) or walkable humps (140).

12. The use of a production line according to one or more of the previous claims for realizing absorbent products for sanitary use.

13. A method for realizing a production line adapted to realize absorbent products for sanitary use, the method comprising a step of realizing said production line by assembling at least two or more manufacturing units (mod1, mod2,..modn) in succession to each other in accordance with a predetermined combination and by fitting said at least two or more manufacturing units (mod1, mod2,..modn) with one or more relative panels (11) equipped with one or more operative apparatuses (60) for executing at least one manufacturing process along said production line, and wherein said step of realization comprises a subphase of equipping said one or more manufacturing units (mod1, mod2,..modn) with at least one structural support frame (52, 53, 54) of standard type, each production line being obtainable by combining in a modular way together, according to any combination, any number of manufacturing units of said two or more manufacturing units (mod1, mod2,..modn) each having said at least one standardized structural support frame (52, 53, 54) and one or more panels (11) standard, so that said panels are part of a group of standard panels each having a predetermined configuration different from the other panels of said group, said standard panels of the group being compatible with the standardized structural support frames belonging to said manufacturing units so that any combination of standard panels belonging to said group of standard panels is applicable in a removable way to each of said manufacturing units.

14. The method, according to claim 13, wherein, in case of need of modification of said production line, there is provided the step of removing one or more manufacturing units (mod1, mod2,..modn) of said two or more manufacturing units (mod1, mod2,..modn) and the relative replacement with one or more manufacturing units (mod1, mod2,..modn) each comprising at least one standardized structural support frame (52, 53, 54).

## Patentansprüche

1. Produktionslinie für absorbierende Produkte für den Hygienegebrauch, wobei die Linie Folgendes umfasst:
- mindestens zwei oder mehrere Fertigungseinheiten (mod1, mod2, ...modn), die nacheinander angeordnet sind, um die Produktionslinie zu bilden,
**dadurch gekennzeichnet, dass**
- jede Fertigungseinheit (mod1, mod2, ... modn) mindestens eine oder mehrere Platten (11) umfasst, wobei jede Platte (11) mit einer oder mehreren Betriebsvorrichtungen (60) ausgestattet ist, die mit der Platte (11) in entfernbarer Weise verbunden werden können, um mindestens einen Fertigungsprozess entlang der Produktionslinie auszuführen, und wobei jede Fertigungseinheit (mod1, mod2, ... modn) ferner mindestens einen tragenden Stützrahmen (52, 53, 54) umfasst, mit dem die mindestens eine oder die mehreren Platten (11) in entfernbarer Weise verbunden sind;
- der mindestens eine tragende Stützrahmen (52, 53, 54) der mindestens zwei oder der mehreren Fertigungseinheiten (mod1, mod2, ... modn) vom Standardtyp ist, sodass jede Produktionslinie erhalten werden kann, indem eine beliebige Anzahl von Fertigungseinheiten (mod1, mod2, ... modn), die jeweils den mindestens einen standardisierten tragenden Stützrahmen (52, 53, 54) umfassen, gemäß einer beliebigen Kombination in modularer Weise miteinander kombiniert wird,
- und wobei die mindestens eine oder die mehreren Platten (11) Standard ist/sind, sodass die Platten Teil einer Gruppe von Standardplatten sind, die jeweils eine vorbestimmte Auslegung aufweisen, die sich von den anderen Platten der Gruppe unterscheidet, wobei die Standardplatten der Gruppe mit den zu den Fertigungseinheiten gehörenden standardisierten tragenden Stützrahmen kompatibel sind, sodass jede beliebige, zu der Gruppe von Standardplatten gehörende Kombination von Standardplatten in entfernbarer Weise an jeder der Fertigungseinheiten angebracht werden kann.

2. Produktionslinie nach Anspruch 1, wobei mindestens ein Teil der elektronischen Komponenten und/oder der hydraulischen Komponenten und/oder der pneumatischen Komponenten an der Platte (11) und/oder an dem mindestens einen tragenden Stützrahmen (52, 53, 54) platziert ist.

3. Produktionslinie nach Anspruch 1 oder 2, wobei die Standardisierung des mindestens einen tragenden Stützrahmens (52, 53, 54) eine geometrische Standardisierung ist.

4. Produktionslinie nach einem oder mehreren der vorhergehenden Ansprüche, die eine Kombination der mindestens zwei Fertigungseinheiten (mod1, mod2, ... modn) umfasst, die gleich sind und alle denselben tragenden Standardstützrahmen (52, 53, 54) aufweisen, oder alternativ die mindestens zwei Fertigungseinheiten (mod1, mod2, ... modn) umfasst, die sich voneinander unterscheiden und jeweils den mindestens einen tragenden Stützrahmen (52, 53, 54) aufweisen, der jeweils unterschiedlich ist, aber stets vom Standardtyp ist.

5. Produktionslinie nach einem oder mehreren der vorhergehenden Ansprüche, wobei die zwei oder die mehreren Fertigungseinheiten (mod1, mod2, ... modn) ausgelegt sind, um das Anbringen einer Schutzbarriere (Ps1, Ps2, ... Psn) entlang der Längsentwicklungsrichtung der Produktionslinie zu ermöglichen, wobei die Schutzbarriere (Ps1, Ps2, ... Psn) in einem Abstand vom vorderen Teil (54) der Produktionslinie so angeordnet ist, dass sie einen Betriebskorridor (100) bildet, auf den ein Bediener und/oder eine weitere Maschine zugreifen und/oder in den sie gelangen können, wobei die Schutzbarriere vorzugsweise an einem Teil des tragenden Stützrahmens (52, 53, 54) der Fertigungseinheit (mod1, mod2, .. modn) und/oder am Boden befestigt ist.

6. Produktionslinie nach Anspruch 5, wobei die Schutzbarriere (Ps1, Ps2, ... Psn) aus einer Vielzahl von Platten (Ps1, Ps2, ... Psn) besteht, die in gleitender Folge zueinander zwischen einer zusammengeschobenen Position, in der sie eine oder mehrere vorhergehende und/oder nachfolgende Platten überlappen, um eine Öffnung zu erzeugen, und einer auseinandergezogenen Position, in der sie diese Öffnung zumindest teilweise verschließen, platziert sind.

7. Produktionslinie nach Anspruch 5 oder 6, wobei die Platten (Ps1, Ps2, ... Psn) aus Kunststoff und/oder durchsichtigem Material bestehen, damit man durch sie hindurchsehen kann.

8. Produktionslinie nach einem oder mehreren der vorhergehenden Ansprüche, umfassend einen automatisierten Roboter (110), der ausgelegt ist, um mindestens eine der mindestens zwei oder mehreren Fertigungseinheiten (mod1, mod2, ... modn) der Produktionslinie entlang der Produktionslinie zu reinigen.

9. Produktionslinie nach Anspruch 8, wobei der automatisierte Roboter (110) an dem durch die Schutzbarriere (Ps1, Ps2, ... Psn) begrenzten Betriebskorridor (100) platziert ist.

10. Produktionslinie nach Anspruch 8 oder 9,
wobei der Roboter (110) entlang einer Führung bewegbar ist, die in der Nähe der zwei oder mehreren Fertigungseinheiten (mod1, mod2, ... modn) ausgebildet ist.

11. Produktionslinie nach einem oder mehreren der vorhergehenden Ansprüche, umfassend eine oder mehrere elektrische Schalttafeln (120) und jeweilige elektrische Verdrahtungen, wobei die elektrischen Schalttafeln (120) in einem bestimmten Abstand von den zwei oder mehreren Fertigungseinheiten (mod1, mod2, ... modn) entlang der Fertigungsentwicklungsrichtung der Produktionslinie in einem bestimmten Abstand von der Rückseite (52) der Produktionslinie derart angeordnet sind, dass ein zweiter Durchgangskorridor (130) gebildet wird, der durch die eine oder die mehreren elektrischen Schalttafeln (120) und durch die Rückseite (52) der Produktionslinie begrenzt wird, wobei die elektrischen Verdrahtungen der einen oder der mehreren elektrischen Schalttafeln (120) vorzugsweise durch Kabelkanäle (140) oder begehbare Abdeckungen (140) abgedeckt sind.

12. Verwendung einer Produktionslinie nach einem oder mehreren der vorhergehenden Ansprüche zur Herstellung von absorbierenden Produkten für den Hygienegebrauch.

13. Verfahren zur Herstellung einer Produktionslinie, die geeignet ist, um absorbierende Produkte für den Hygienegebrauch herzustellen, wobei das Verfahren einen Schritt zum Herstellen der Produktionslinie umfasst, indem mindestens zwei oder mehrere Fertigungseinheiten (mod1, mod2, ... modn) nacheinander gemäß einer vorbestimmten Kombination zusammengebaut werden und indem die mindestens zwei oder mehreren Fertigungseinheiten (mod1, mod2, ... modn) mit einer oder mehreren relativen Platten (11), die mit einer oder mehreren Betriebsvorrichtungen (60) zum Ausführen mindestens eines Fertigungsprozesses entlang der Produktionslinie ausgestattet sind, versehen werden, und wobei der Schritt zum Herstellen eine Unterphase zum Ausstatten der einen oder mehreren Fertigungseinheiten (mod1, mod2, ... modn) mit mindestens einem tragenden Stützrahmen (52, 53, 54) vom Standardtyp umfasst, wobei jede Produktionslinie erhalten werden kann, indem eine beliebige Anzahl von Fertigungseinheiten der zwei oder mehreren Fertigungseinheiten (mod1, mod2, ... modn), die jeweils den mindestens einen standardisierten tragenden Stützrahmen (52, 53, 54) sowie eine oder mehrere Standardplatten (11) aufweisen, gemäß einer beliebigen Kombination in modularer Weise miteinander kombiniert wird, sodass die Platten Teil einer Gruppe von Standardplatten sind, die jeweils eine vorbestimmte Auslegung aufweisen, die sich von den anderen Platten der Gruppe unterscheidet, wobei die Standardplatten der Gruppe mit den zu den Fertigungseinheiten gehörenden standardisierten Stützrahmen kompatibel sind, sodass jede beliebige, zu der Gruppe von Standardplatten gehörende Kombination von Standardplatten in entfernbarer Weise an jeder der Fertigungseinheiten angebracht werden kann.

14. Verfahren nach Anspruch 13, wobei, sollte die Notwendigkeit einer Änderung der Produktionslinie bestehen, der Schritt zum Entfernen einer oder mehrerer Fertigungseinheiten (mod1, mod2, ... modn) der zwei oder mehreren Fertigungseinheiten (mod1, mod2, ... modn) und der entsprechende Ersatz mit einer oder mehreren Fertigungseinheiten (mod1, mod2, ... modn), die jeweils mindestens einen standardisierten tragenden Stützrahmen (52, 53, 54) umfassen, bereitgestellt wird.

## Revendications

1. Ligne de production de produits absorbants à usage sanitaire, ladite ligne comprenant :
- au moins deux ou plusieurs unités de fabrication (mod1, mod2, ... modn) disposées les unes à la suite des autres pour former ladite ligne de production,
- **caractérisée en ce que** :
- chaque unité de fabrication (mod1, mod2, .. modn) comprend au moins un ou plusieurs panneaux (11), chaque panneau (11) étant équipé d'un ou plusieurs appareils fonctionnels (60) pouvant être reliés au panneau (11) de manière amovible pour exécuter au moins un processus de fabrication le long de ladite ligne de production, et chaque unité de fabrication (mod1, mod2, .. modn) comprenant en outre au moins un cadre de support structurel (52, 53, 54) auquel le ou les panneaux (11) sont reliés de manière amovible ;
- ledit cadre de support structurel (52, 53, 54) desdites au moins deux ou plusieurs unités de fabrication (mod1, mod2, .. modn) est de type standard de sorte que chaque ligne de production peut être obtenue en combinant de manière modulaire, selon n'importe quelle combinaison, un nombre quelconque d'unités de fabrication (mod1, mod2, .. modn) comprenant chacune ledit cadre de support structurel (52, 53, 54) standardisé ;
- et ledit ou lesdits panneaux (11) étant standard, de sorte que lesdits panneaux font partie d'un groupe de panneaux standard ayant chacun une configuration prédéterminée différente des autres panneaux dudit groupe, lesdits panneaux standard du groupe étant compatibles avec les cadres de support structurel standardisés appartenant auxdites unités de fabrication, de sorte que toute combinaison de panneaux standard appartenant audit groupe de panneaux standard est applicable de manière amovible à chacune desdites unités de fabrication.

2. Ligne de production selon la revendication 1, au moins une partie des composants électroniques et/ou des composants hydrauliques et/ou des composants pneumatiques étant placée au niveau dudit panneau (11) et/ou sur ledit cadre de support structurel (52, 53, 54).

3. Ligne de production selon la revendication 1 ou 2, ladite standardisation dudit cadre de support structurel (52, 53, 54) étant une standardisation géométrique.

4. Ligne de production, selon l'une ou plusieurs des revendications précédentes, qui comprend une combinaison desdites au moins deux unités de fabrication (mod1, mod2, .. modn) égales entre elles et comportant toutes le même cadre de support structurel standard (52, 53, 54) ou, alternativement, comprend lesdites au moins deux unités de fabrication (mod1, mod2, .. modn) différentes les unes des autres et comportant chacune ledit cadre de support structurel (52, 53, 54) différent des autres mais toujours de type standard.

5. Ligne de production, selon l'une ou plusieurs des revendications précédentes, lesdits deux ou plusieurs unités de fabrication (mod1, mod2, .. modn) étant configurées pour permettre l'application d'une barrière de protection (Ps1, Ps2, ..). Psn) le long de la direction de développement longitudinal de ladite ligne de production, ladite barrière de protection (Ps1, Ps2, ... Psn) étant placée à une certaine distance de la partie avant (54) de la ligne de production de manière à former un couloir fonctionnel (100) auquel un opérateur et/ou une machine supplémentaire peut accéder et/ou entrer ; de préférence, la barrière de protection étant fixée à une partie du cadre de support structurel (52, 53, 54) de ladite unité de fabrication (mod1, mod2, .. modn) et/ou au sol.

6. Ligne de production selon la revendication 5, ladite barrière de protection (Ps1, Ps2, .... Psn) étant composée d'une pluralité de panneaux (Ps1, Ps2, .... Psn) placés les uns à la suite des autres de façon coulissante entre une position tassée, dans laquelle ils chevauchent un ou plusieurs panneaux précédents et/ou suivants pour générer une ouverture, et une position déployée dans laquelle ils ferment ladite ouverture au moins partiellement.

7. Ligne de production selon la revendication 5 ou 6, lesdits panneaux (Ps1, Ps2, .... Psn) étant en plastique et/ou en matériau transparent pour permettre de voir à travers.

8. Ligne de production, selon l'une ou plusieurs des revendications précédentes, comprenant un robot automatisé (110) configuré pour nettoyer au moins une desdites deux ou plusieurs unités de fabrication (mod1, mod2, .. modn) de la ligne de production, le long de la ligne de production.

9. Ligne de production selon la revendication 8, ledit robot automatisé (110) étant placé au niveau du couloir fonctionnel (100) délimité par ladite barrière de protection (Ps1, Ps2, .. Psn).

10. Ligne de production selon la revendication 8 ou 9,
le robot (110) étant mobile le long d'un guide obtenu à proximité desdites deux ou plusieurs unités de fabrication (mod1, mod2, .. modn).

11. Ligne de production, selon l'une ou plusieurs des revendications précédentes, comprenant un ou plusieurs panneaux électriques (120) et les câblages électriques correspondants, lesdits panneaux électriques (120) étant placés à une certaine distance desdites deux ou plusieurs unités de fabrication (mod1, mod2, .... modn) le long de la direction de développement de la fabrication de ladite ligne de production à une certaine distance de la face arrière (52) de la ligne de production, de manière à former un second couloir de passage (130) délimité par ledit ou lesdits panneaux électriques (120) et par la face arrière (52) de la ligne de production ; de préférence, les câblages électriques dudit ou desdits panneaux électriques (120) sont recouverts par des chemins de roulement (140) ou des buttes accessibles (140).

12. Utilisation d'une ligne de production selon l'une ou plusieurs des revendications précédentes pour la réalisation de produits absorbants à usage sanitaire.

13. Procédé de réalisation d'une ligne de production adaptée pour réaliser des produits absorbants à usage sanitaire, le procédé comprenant une étape de réalisation de ladite ligne de production en assemblant au moins deux ou plusieurs unités de fabrication (mod1, mod2, .. modn) les unes à la suite des autres selon une combinaison prédéterminée et en adaptant lesdites au moins deux ou plusieurs unités de fabrication (mod1, mod2, .. modn) avec un ou plusieurs panneaux relatifs (11) équipés d'un ou plusieurs appareils fonctionnels (60) pour exécuter au moins un processus de fabrication le long de ladite ligne de production, et ladite étape de réalisation comprenant une sous-phase consistant à équiper ladite ou lesdites unités de fabrication (mod1, mod2, ... modn) d'au moins un cadre de support structurel (52, 53, 54) de type standard, chaque ligne de production pouvant être obtenue en combinant de manière modulaire, selon n'importe quelle combinaison, n'importe quel nombre d'unités de fabrication desdites deux ou plusieurs unités de fabrication (mod1, mod2, ... modn) comportant chacune ledit cadre de support structurel standardisé (52, 53, 54) et un ou plusieurs panneaux (11) standard, de sorte que lesdits panneaux font partie d'un groupe de panneaux standard ayant chacun une configuration prédéterminée différente des autres panneaux dudit groupe, lesdits panneaux standard du groupe étant compatibles avec les cadres de support structurel standardisés appartenant auxdites unités de fabrication de sorte que toute combinaison de panneaux standard appartenant audit groupe de panneaux standard est applicable de manière amovible à chacune desdites unités de fabrication.

14. Procédé selon la revendication 13, dans lequel, en cas de nécessité de modification de ladite ligne de production, il est prévu l'étape consistant à retirer une ou plusieurs unités de fabrication (mod1, mod2, .. modn) desdites deux ou plusieurs unités de fabrication (mod1, mod2, .. modn) et le remplacement relatif par une ou plusieurs unités de fabrication (mod1, mod2, .. modn) comprenant chacune au moins un cadre de support structurel standardisé (52, 53, 54).
